# EUROPEAN PATENT SPECIFICATION

(11) **EP 3 002 286 B1**
(45) Date of publication and mention of the grant of the patent: **25.04.2018**
(21) Application number: 14883332.0
(22) Date of filing: 27.10.2014
(51) Int. Cl.: C07D 487/04, A61K 31/5025, A61P 25/08

(54) **PREPARATION METHOD FOR POLYMORPHIC 6-(4-CHLOROPHENOXY)-TETRAZOLO[5,1-A]PHTHALAZINE AND USE THEREOF**
HERSTELLUNGSVERFAHREN FÜR POLYMORPHES 6-(4-CHLOROPHENOXY)-TETRAZOLO[5,1-A]PHTHALAZIN UND VERWENDUNG DAVON
PROCÉDÉ DE PRÉPARATION DE 6-(4-CHLOROPHÉNOXY)-TÉTRAZOLO[5,1-A]PHTALAZINE POLYMORPHE ET SON UTILISATION

(30) Priority: 21.02.2014 CN 201410059768
(43) Date of publication of application: 06.04.2016
(73) Proprietor: Jilin Yinglian Shangde Science and Technology Development Co., Ltd., Jilin 130033 (CN)
(72) Inventor: QUAN, Zheshan, Jilin 130033 (CN); LI, Wei, Jilin 130033 (CN); DONG, Fangyan, Jilin 130033 (CN); YU, Yingzhou, Jilin 130033 (CN)
(74) Representative: Nordmeyer, Philipp Werner
(86) International application number: PCT/CN2014/000946
(87) International publication number: WO 2015/123801

(56) References cited:
- CN-A- 101 676 287
- CN-A- 103 819 477
- SUN ET AL.: "Synthesis and primary anticonvulsant activity evaluation of 6-alkyoxyl-tetrazolo[5,1-a]phthalazine derivatives", ARZNEIMITTELFORSCHUNG, vol. 60, no. 6, 2010, pages 289-292, XP1526594,
- BYRN ET AL.: "PHARMACEUTICAL SOLIDS: A STRATEGIC APPROACH TO REGULATORY CONSIDERATIONS", PHARM. RES., vol. 12, no. 7, 1 July 1995 (1995-07-01), pages 945-954, XP000996386, ISSN: 0724-8741, DOI: 10.1023/A:1016241927429
- BRITTAIN: "SPECTRAL METHODS FOR THE CHARACTERIZATION OF POLYMORPHS AND SOLVATES", J. PHARM. SCI., vol. 86, no. 4, 1 April 1997 (1997-04-01), pages 405-412, XP001204463, ISSN: 0022-3549, DOI: 10.1021/JS960238E
- BYRN ET AL.: "Solid-State Chemistry of Drugs", 1999, SSCI INC, WEST LAFAYETTE, XP002588305, ISBN: 978-0-9670671-0-0 pages 82-85, * Chapter 5.2 Differential Scanning Calorimetry. Cited as common general knowledge. *

## Description

### Field of the Invention

This invention belongs to the field of chemical technology, and relates to processes for preparing polymorphic forms of 6-(4-chlorophenoxy)-tetrazolo[5,1-*a*]phthalazine, and use thereof.

### Background of the Invention

It has been found during the current research on crystals that a same compound can exist in two or more crystalline states. When a compound of the same molecular structure exists in different crystal forms, it may have a different bioavailability, solubility, dissolution rate, melting point, color, filterability, density, mobility, mechanical stability, etc., and such physicochemical properties or processibilities sometimes directly affect drug safety or efficacy. Therefore, study of crystal forms and control thereof became a research focus during drug research and development.

Study of crystal forms includes two stages, i.e., discovery of crystals and optimization of crystal forms. During the stage of discovery of crystals, various crystallization means, such as melt crystallization, volatilization of solutions, rapid cooling, and suspension methods, are mainly employed, which affect external factors of the drug crystallization with changes in crystallization conditions, such as solvent, temperature, speed, ratio between solvents for suspension, and etc. A high-throughout sample preparation platform can be employed to run hundreds of crystallization tests simultaneously, and micro-scale sample preparation techniques as well as analyzing and testing means are utilized to prepare and find new crystal forms. During the stage of optimization of crystal forms, scale-up of processes and investigation of preparation conditions for a new crystal form are carried out, and the crystal form can be characterized utilizing a variety of solid characterization techniques, such as X-ray diffraction, solid state nuclear magnetic resonance, Raman spectroscopy, infrared spectroscopy, and etc. Furthermore, a physicochemical property study of the crystal form is carried out utilizing DSC, TG, DVS, HPLC, and etc., so as to compare the properties of different crystal forms, such as hygroscopicity, chemical stability, physical state stability, processibility, and etc. Eventually, the most preferred solid form is chosen for further development.

In the prior Chinese patent application No. CN200810165734.X, a series of tetrazolo[5,1-a]phthalazine derivatives and use thereof for the manufacturing of an anti-epileptic medicament were described. Specifically, 6-(4-chlorophenoxy)-tetrazolo[5,1-a]phthalazine was described therein.

The chemical structure of 6-(4-chlorophenoxy)-tetrazolo[5,1-a]phthalazine is as shown in Formula I below.

Currently, investigation of 6-(4-chlorophenoxy)-tetrazolo[5,1-a]phthalazine as an anti-epileptic drug is still in an early stage of drug development, while there has been no study report on crystal forms thereof. Therefore, it is of great importance to study the crystal forms thereof so as to obtain the most preferred solid form for further development.

### Summary of the invention

To resolve the problems as set forth above, this invention provides a process for preparing polymorphic form C of 6-(4-chlorophenoxy)-tetrazolo[5,1-*a*]phthalazine and use thereof, wherein 6-(4-chlorophenoxy)-tetrazolo[5,1-*a*]phthalazine is a compound as shown in Formula I:

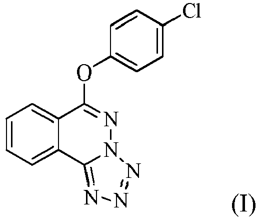

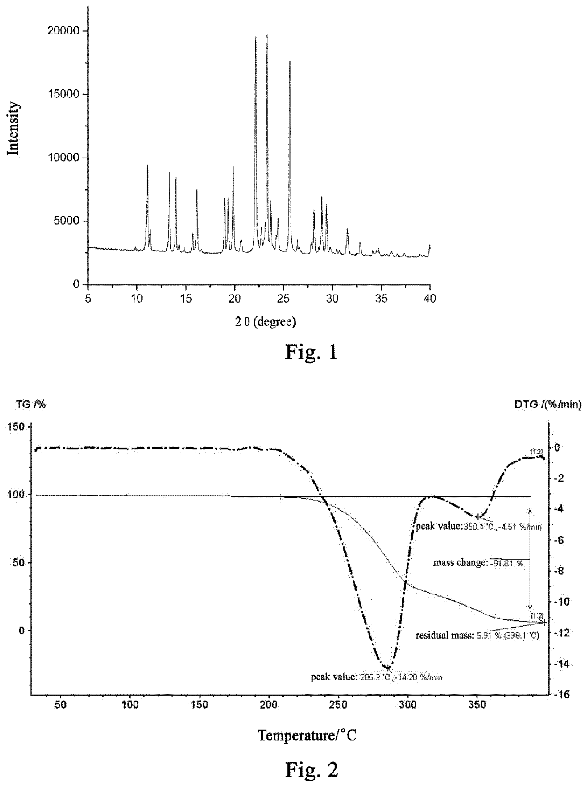

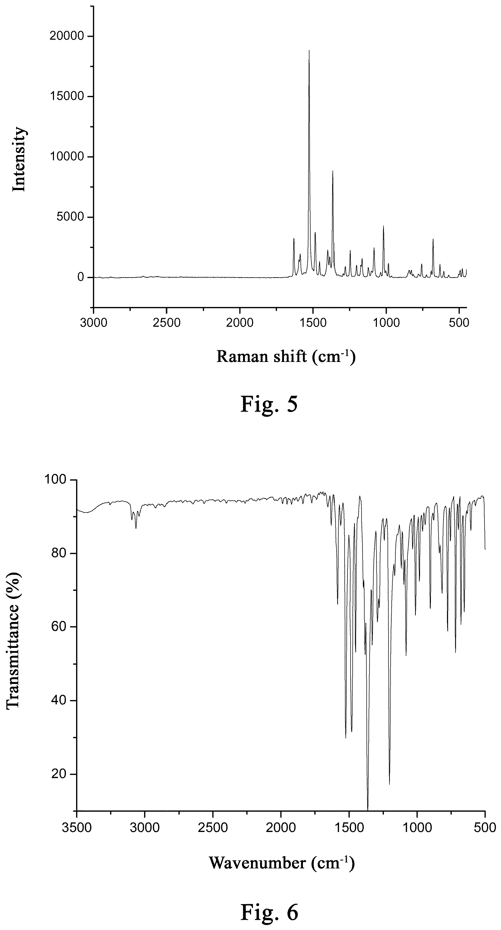

Described is a crystalline form A of 6-(4-chlorophenoxy)-tetrazolo[5,1-*a*]phthalazine, characterized by one or more of the following properties:
i) an X-ray powder diffraction pattern having characteristic peaks expressed in degrees 2θ at 10.99, 11.29, 13.24, 13.92, 15.67, 16.08, 18.90, 19.25, 19.81, 22.06, 22.68, 23.28, 23.66, 24.22, 24.40, 25.62, 26.36, 27.75, 28.01, 28.84, 29.34, 31.51, 32.75, 33.37, 34.47, 39.91, and an X-ray powder diffraction pattern in accordance with Figure 1;
ii) a differential scanning calorimetry thermogram having a characteristic peak at 205.4 °C;
iii) an FT-IR spectrum in accordance with Figure 6.

Described herein is also process for preparing the crystalline form A of 6-(4-chlorophenoxy)-tetrazolo[5,1-*a*]phthalazine, comprising the following steps:
adding 6-(4-chlorophenoxy)-tetrazolo[5,1-*a*]phthalazine into a first volume of a solvent in the proportion of about 1:25 g/ml and at the boiling point temperature of the solvent, then further adding the solvent slowly until 6-(4-chlorophenoxy)-tetrazolo[5,1-*a*]phthalazine is dissolved totally, then cooling down to 0 °C rapidly, filtering precipitated crystals and washing with a second volume of the said solvent, the second volume being 10% of the first volume, and then drying under a normal or reduced pressure at a temperature in a range of from room temperature to 100 °C to obtain a crystalline form A of 6-(4-chlorophenoxy)-tetrazolo[5,1-*a*]phthalazine;
wherein the solvent is one selected from acetonitrile, propanone, ethyl acetate, tetrahydrofuran, methylene dichloride and toluene.

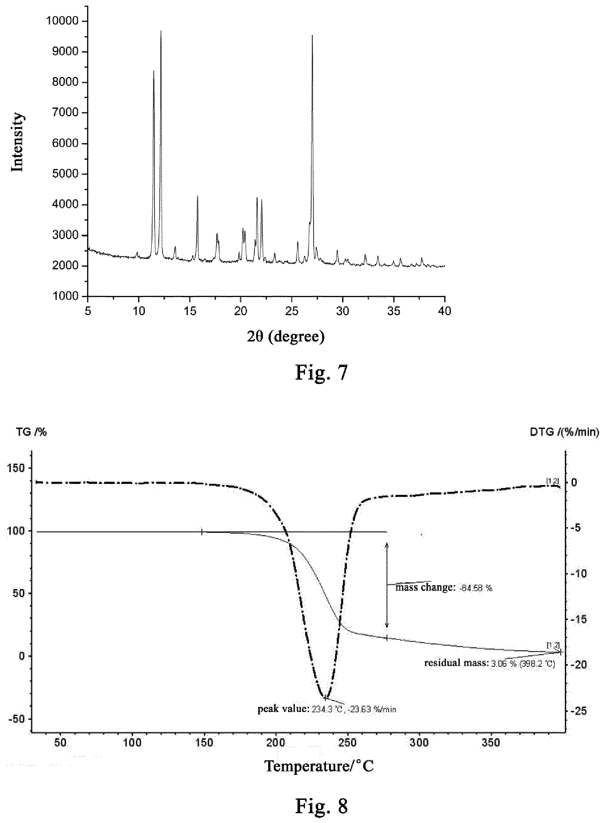

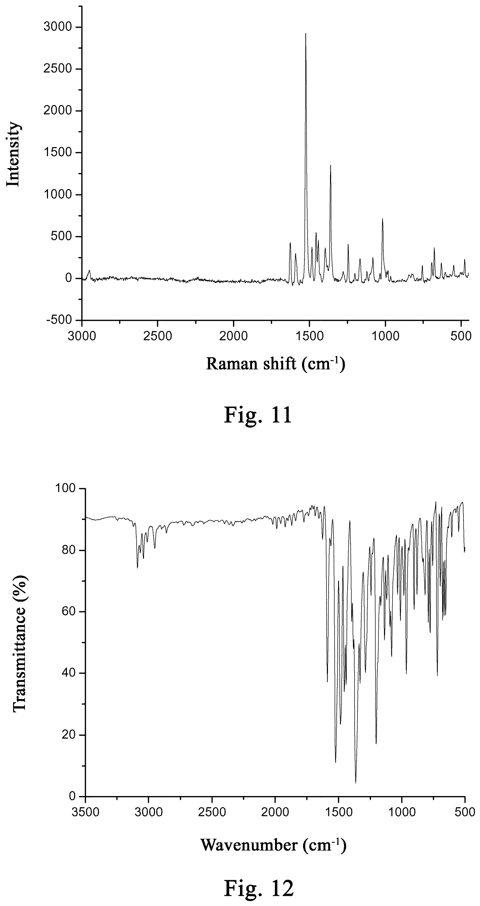

Further described is a crystalline form B of 6-(4-chlorophenoxy)-tetrazolo[5,1-*a*]phthalazine, characterized by one or more of the following properties:
i) an X-ray powder diffraction pattern having characteristic peaks expressed in degrees 2θ at about 9.84, 11.47, 12.18, 13.57, 15.28, 15.76, 17.68, 19.84, 20.23, 20.41, 21.38, 21.59, 22.06, 23.31, 25.59, 26.27, 26.74, 27.01, 27.39, 27.84, 29.46, 30.26, 32.19, 33.43, 34.97, 35.65, 37.75, and an X-ray powder diffraction pattern in accordance with Figure 7;
ii) a differential scanning calorimetry thermogram having characteristic peaks at 163.9 °C and 165.5 °C;
iii) an FT-IR spectrum in accordance with Figure 12.

Also described is a process for preparing the crystalline form B of 6-(4-chlorophenoxy)-tetrazolo[5,1-*a*]phthalazine, comprising the following steps:
suspending 6-(4-chlorophenoxy)-tetrazolo[5,1-*a*]phthalazine with a first volume of methanol as a solvent in the proportion of about 25:1 g/L and at a temperature of 50 °C, mixing the resultant suspension with a magnetic stirrer at a rotational speed of 60-600 rpm for two days, then filtering the suspension and washing with a second volume of methanol, the second volume being 10% of the first volume, and then drying at room temperature to obtain a crystalline form B of 6-(4-chlorophenoxy)-tetrazolo[5,1-*a*]phthalazine.

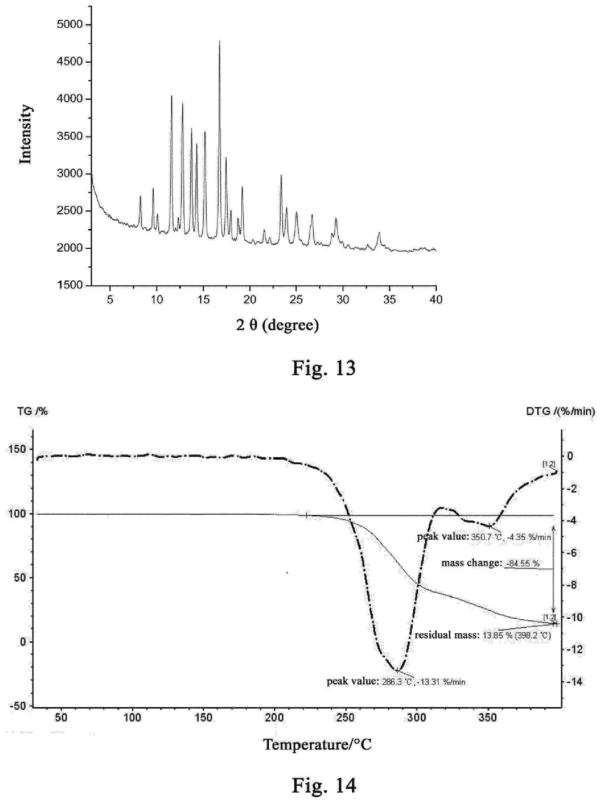

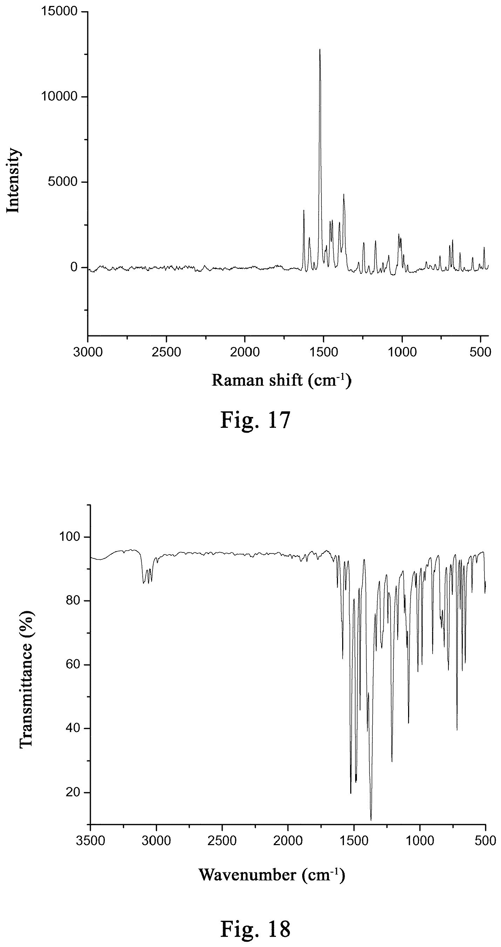

This invention provides a crystalline form C of 6-(4-chlorophenoxy)-tetrazolo[5,1-*a*]phthalazine, characterized by one or more of the following properties:
i) an X-ray powder diffraction pattern having characteristic peaks expressed in degrees 2θ at about 8.24, 9.60, 10.07, 11.58, 12.27, 12.74, 13.69, 14.25, 15.17, 16.73, 17.45, 17.92, 18.72, 19.16, 20.35, 21.53, 22.12, 23.37, 23.96, 25.05, 26.62, 28.78, 29.23, 33.84, and an X-ray powder diffraction pattern in accordance with Figure 13;
ii) a differential scanning calorimetry thermogram having a characteristic peak at 205.6 °C;
iii) an FT-IR spectrum in accordance with Figure 18.

This invention also provides a process for preparing the crystalline form C of 6-(4-chlorophenoxy)-tetrazolo[5,1-*a*]phthalazine, comprising the following steps:
adding 6-(4-chlorophenoxy)-tetrazolo[5,1-*a*]phthalazine into a solvent in the proportion of about 15:1 g/L, mixing homogeneously until 6-(4-chlorophenoxy)-tetrazolo[5,1-*a*]phthalazine is dissolved, then volatilizing slowly at a temperature of 25 °C or 50 °C until dry, and then collecting solid to obtain a crystalline form C of 6-(4-chlorophenoxy)-tetrazolo[5,1-*a*]phthalazine;
wherein the solvent along with the corresponding temperature for the volatilizing step is one selected from methyl ethyl ketone along with 25 °C, nitromethane along with 25 °C, methylbenzene along with 25 °C, propanone along with 50 °C, and acetonitrile along with 50 °C.

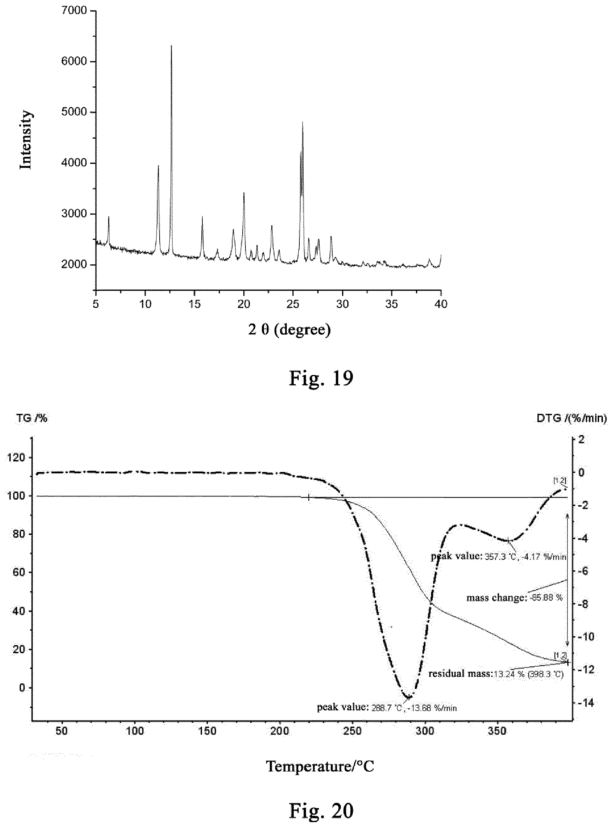

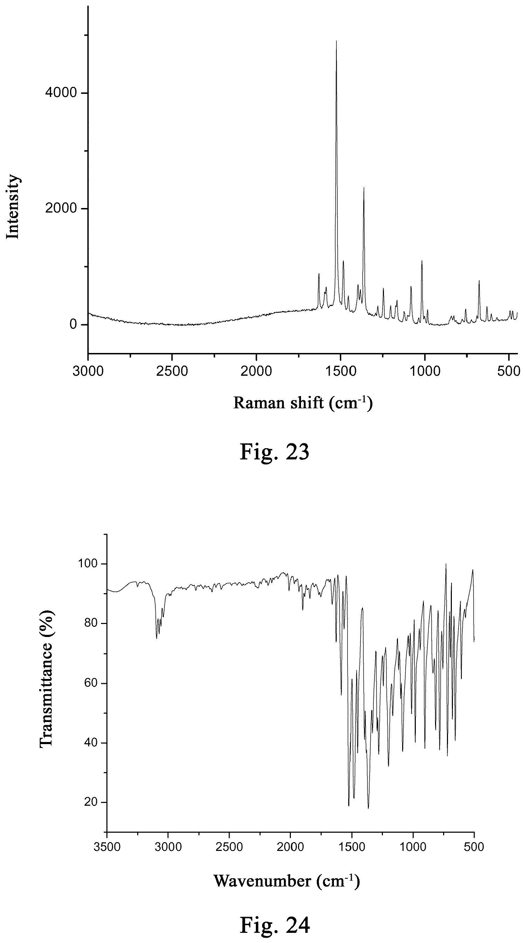

Further described is a crystalline form D of 6-(4-chlorophenoxy)-tetrazolo[5,1-a]phthalazine, characterized by one or more of the following properties:
i) an X-ray powder diffraction pattern having characteristic peaks expressed in degrees 2θ at about 6.32, 11.35, 12.68, 15.85, 18.93, 19.07,20.02,21.35,22.89,23.63,25.79,25.00, 26.59, 27.30, 27.60, 28.84, 32.10, 33.58, 38.79, and an X-ray powder diffraction pattern in accordance with Figure 19;
ii) a differential scanning calorimetry thermogram having a characteristic peak at 205.9 °C;
iii) an FT-IR spectrum in accordance with Figure 24.

Described is also a process for preparing the crystalline form D of 6-(4-chlorophenoxy)-tetrazolo[5,1-*a*]phthalazine, comprising the following steps:
adding 6-(4-chlorophenoxy)-tetrazolo[5,1-*a*]phthalazine into a mixed solvent in the proportion of about 15:2 g/L, mixing homogeneously until 6-(4-chlorophenoxy)-tetrazolo[5,1-*a*]phthalazine is dissolved, then volatilizing slowly at a temperature of 50 °C until dry, and then collecting solid to obtain a crystalline form D of 6-(4-chlorophenoxy)-tetrazolo[5,1-*a*]phthalazine;
wherein the mixed solvent is one selected from a mixture of nitromethane and water at a volume ratio of 2:1, a mixture of nitromethane and n-hexane at a volume ratio of 1:1, and a mixture of nitromethane and heptane at a volume ratio of 1:1.

This invention also provides use of the crystalline form C of 6-(4-chlorophenoxy)-tetrazolo[5,1-*a*]phthalazine for the manufacturing of an anti-epileptic medicament.

This invention also provides a pharmaceutical composition comprising the crystalline form C of 6-(4-chlorophenoxy)-tetrazolo[5,1-*a*]phthalazine, and a pharmaceutically acceptable excipient. The invention further provides crystalline form C of 6-(4-chlorophenoxy)-tetrazolo[5,1-*a*]phthalazine for use in treating epilepsy

Compared to the prior art technologies, this invention has the following advantageous effects: the preparation processes of this invention are simple to operate and have excellent reproducibility such that the desired crystal forms can be obtained steadily, and the novel crystal forms have better water solubility and can exhibit better pharmacological effect of anti-convulsion than the prior crystal forms.

### Brief Description of the Drawings

Figure 1 shows an X-ray powder diffraction (XRPD) pattern for the crystalline form A of 6-(4-chlorophenoxy)-tetrazolo[5,1-*a*]phthalazine in Examples 1 and 2;
Figure 2 shows a thermogravimetric (TG) analysis curve for the crystalline form A of 6-(4-chlorophenoxy)-tetrazolo[5,1-*a*]phthalazine in Examples 1 and 2;

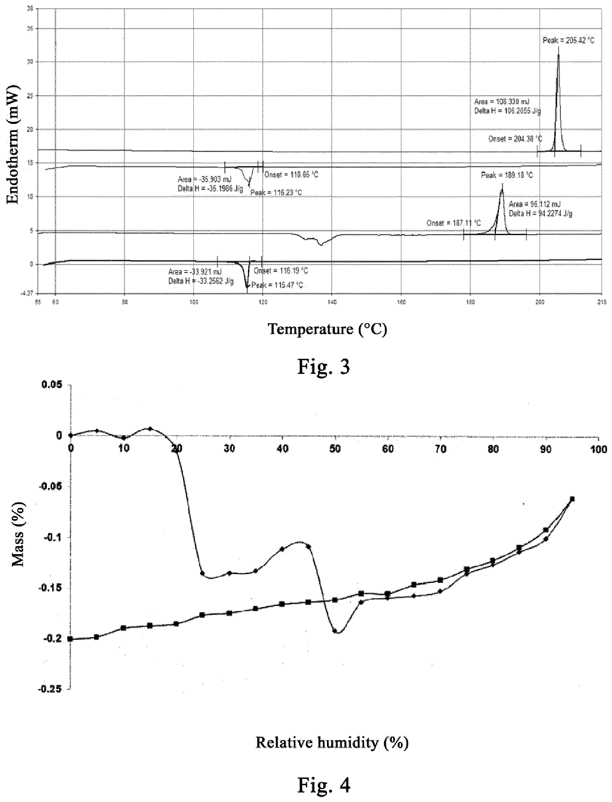

Figure 3 shows a differential scanning calorimetry (DSC) thermogram for the crystalline form A of 6-(4-chlorophenoxy)-tetrazolo[5,1-*a*]phthalazine in Examples 1 and 2;
Figure 4 shows a dynamic vapour sorption (DVS) analysis curve for the crystalline form A of 6-(4-chlorophenoxy)-tetrazolo[5,1-*a*]phthalazine in Examples 1 and 2;
Figure 5 shows a Raman spectrum for the crystalline form A of 6-(4-chlorophenoxy)-tetrazolo[5,1-*a*]phthalazine in Examples 1 and 2;
Figure 6 shows an Infrared (IR) spectrum for the crystalline form A of 6-(4-chlorophenoxy)-tetrazolo[5,1-*a*]phthalazine in Examples 1 and 2;
Figure 7 shows an X-ray powder diffraction (XRPD) pattern for the crystalline form B of 6-(4-chlorophenoxy)-tetrazolo[5,1-*a*]phthalazine in Example 3;
Figure 8 shows a thermogravimetric (TG) analysis curve for the crystalline form B of 6-(4-chlorophenoxy)-tetrazolo[5,1-*a*]phthalazine in Example 3;

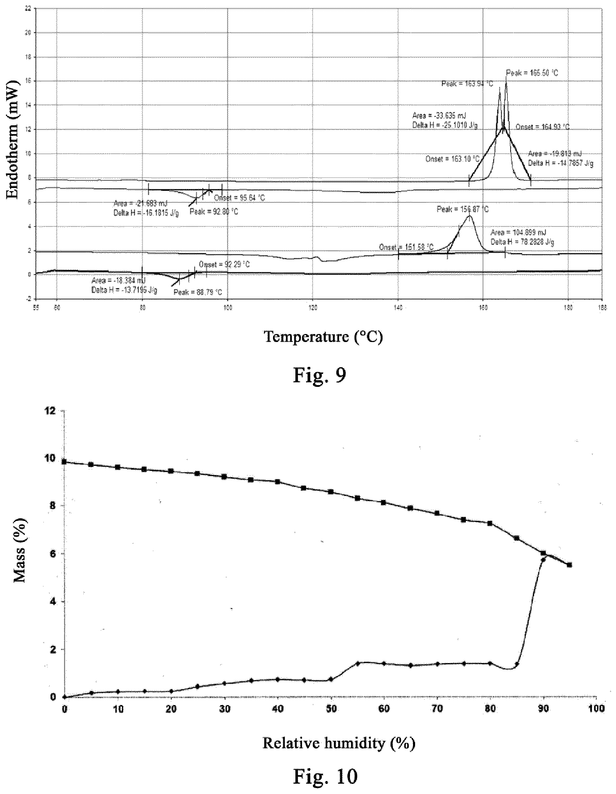

Figure 9 shows a differential scanning calorimetry (DSC) thermogram for the crystalline form B of 6-(4-chlorophenoxy)-tetrazolo[5,1-*a*]phthalazine in Example 3;
Figure 10 shows a dynamic vapour sorption (DVS) analysis curve for the crystalline form B of 6-(4-chlorophenoxy)-tetrazolo[5,1-*a*]phthalazine in Example 3;
Figure 11 shows a Raman spectrum for the crystalline form B of 6-(4-chlorophenoxy)-tetrazolo[5,1-*a*]phthalazine in Example 3;
Figure 12 shows an Infrared (IR) spectrum for the crystalline form B of 6-(4-chlorophenoxy)-tetrazolo[5,1-a]phthalazine in Example 3;
Figure 13 shows an X-ray powder diffraction (XRPD) pattern for the crystalline form C of 6-(4-chlorophenoxy)-tetrazolo[5,1-*a*]phthalazine in Examples 4 and 5;
Figure 14 shows a thermogravimetric (TG) analysis curve for the crystalline form C of 6-(4-chlorophenoxy)-tetrazolo[5,1-*a*]phthalazine in Examples 4 and 5;

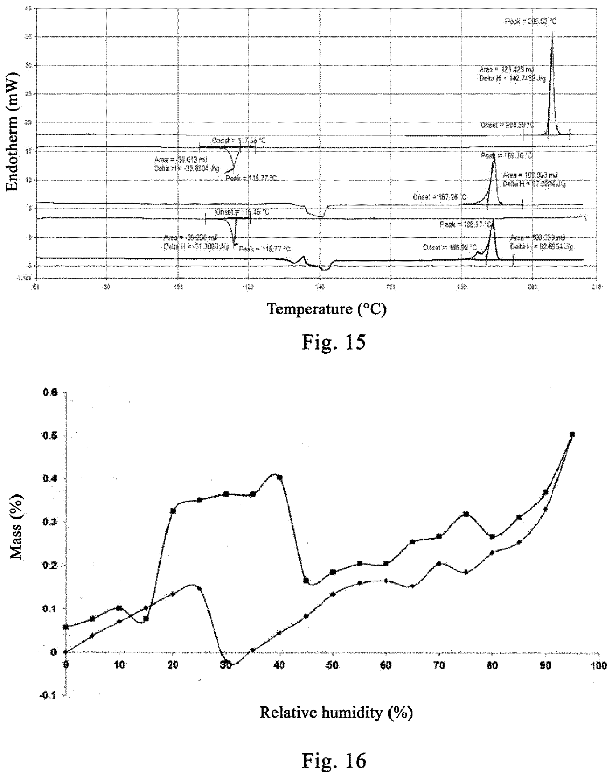

Figure 15 shows a differential scanning calorimetry (DSC) thermogram for the crystalline form C of 6-(4-chlorophenoxy)-tetrazolo[5,1-*a*]phthalazine in Examples 4 and 5;
Figure 16 shows a dynamic vapour sorption (DVS) analysis curve for the crystalline form C of 6-(4-chlorophenoxy)-tetrazolo[5,1-*a*]phthalazine in Examples 4 and 5;
Figure 17 shows a Raman spectrum for the crystalline form C of 6-(4-chlorophenoxy)-tetrazolo[5,1-*a*]phthalazine in Examples 4 and 5;
Figure 18 shows an Infrared (IR) spectrum for the crystalline form C of 6-(4-chlorophenoxy)-tetrazolo[5,1-*a*]phthalazine in Examples 4 and 5;
Figure 19 shows an X-ray powder diffraction (XRPD) pattern for the crystalline form D of 6-(4-chlorophenoxy)-tetrazolo[5,1-*a*]phthalazine in Example 6;
Figure 20 shows a thermogravimetric (TG) analysis curve for the crystalline form D of 6-(4-chlorophenoxy)-tetrazolo[5,1-*a*]phthalazine in Example 6;

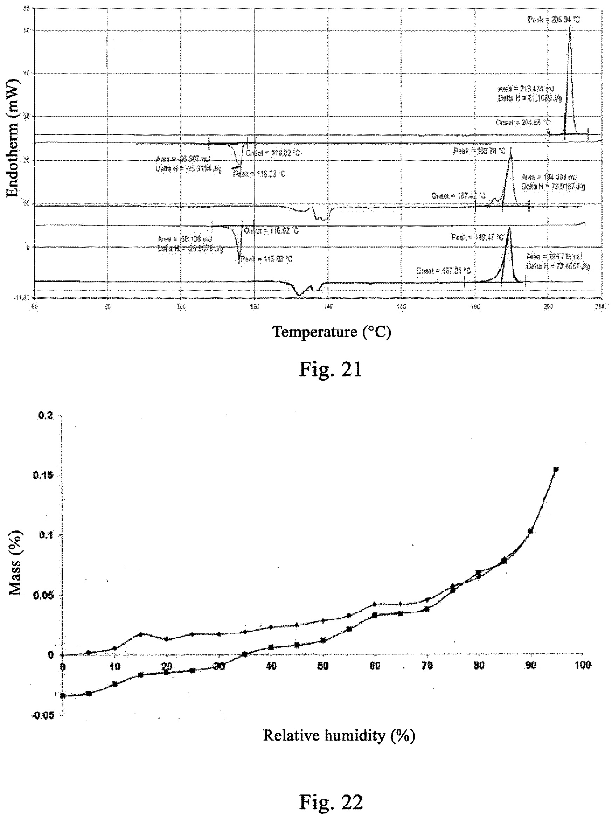

Figure 21 shows a differential scanning calorimetry (DSC) thermogram for the crystalline form D of 6-(4-chlorophenoxy)-tetrazolo[5,1-*a*]phthalazine in Example 6;
Figure 22 shows a dynamic vapour sorption (DVS) analysis curve for the crystalline form D of 6-(4-chlorophenoxy)-tetrazolo[5,1-*a*]phthalazine in Example 6;
Figure 23 shows a Raman spectrum for the crystalline form D of 6-(4-chlorophenoxy)-tetrazolo[5,1-*a*]phthalazine in Example 6;
Figure 24 shows an Infrared (IR) spectrum for the crystalline form D of 6-(4-chlorophenoxy)-tetrazolo[5,1-*a*]phthalazine in Example 6.

### Detailed Description of the Preferred Embodiments

This invention is now described in more detail by way of examples with reference to the figures hereinafter.

### Comparative Example 1: Preparation of a crystalline form A of 6-(4-chlorophenoxy)-tetrazolo[5,1-a]phthalazine

1g of 6-(4-chlorophenoxy)-tetrazolo[5,1-*a*]phthalazine was added into 20ml of acetonitrile as a solvent at 80 °C, followed by slowly adding additional solvent of the same until 6-(4-chlorophenoxy)-tetrazolo[5,1-*a*]phthalazine was dissolved totally, and then the mixture was cooled down to 0 °C rapidly. Precipitated crystals were filtered and collected, followed by washing with a small amount of acetonitrile, and drying at 50°C to give 0.82g of a white crystalline powder (crystalline form A), with a yield of 82%.

### Comparative Example 2: Preparation of a crystalline form A of 6-(4-chlorophenoxy)-tetrazolo[5,1-a]phthalazine

1g of 6-(4-chlorophenoxy)-tetrazolo[5,1-*a*]phthalazine was added into 20ml of propanone as a solvent at 60°C, followed by slowly adding additional solvent of the same until 6-(4-chlorophenoxy)-tetrazolo[5,1-*a*]phthalazine was dissolved totally, and then the mixture was cooled down to 0 °C rapidly. Precipitated crystals were filtered and collected, followed by washing with a small amount of propanone, and drying at 50 °C to give 0.67g of a white crystalline solid (crystalline form A), with a yield of 67%.

### Comparative Example 3: Preparation of a crystalline form B of 6-(4-chlorophenoxy)-tetrazolo[5,1-a]phthalazine

1g of 6-(4-chlorophenoxy)-tetrazolo[5,1-*a*]phthalazine was suspended with 40ml of methanol as a solvent at 50 °C, followed by mixing the resultant suspension with a magnetic stirrer at a rotational speed of 200 rpm for two days, and then the suspension was filtered, and the product obtained was washed with a small amount of methanol, followed by drying at room temperature to give 0.84g of a white crystalline solid (crystalline form B), with a yield of 84%.

### Example 4: Preparation of a crystalline form C of 6-(4-chlorophenoxy)-tetrazolo[5,1-a]phthalazine

1g of 6-(4-chlorophenoxy)-tetrazolo[5,1-*a*]phthalazine was added into 60ml of methyl ethyl ketone as a solvent, followed by mixing homogeneously until 6-(4-chlorophenoxy)-tetrazolo[5,1-a]phthalazine was dissolved. The mixture was volatilized slowly at 25 °C until dry to give 0.95 g of a white floccular solid (crystalline form C), with a yield of 95%.

### Example 5: Preparation of a crystalline form C of 6-(4-chlorophenoxy)-tetrazolo[5,1-a]phthalazine

1g of 6-(4-chlorophenoxy)-tetrazolo[5,1-*a*]phthalazine was added into 60ml of propanone as a solvent, followed by mixing homogeneously until 6-(4-chlorophenoxy)-tetrazolo[5,1-*a*]phthalazine was dissolved, which could be done by heating slightly. The mixture was volatilized slowly at 50 °C until dry to give 0.96 g of a white powdered solid (crystalline form C), with a yield of 96%.

### Comparative Example 6: Preparation of a crystalline form D of 6-(4-chlorophenoxy)-tetrazolo[5,1-a]phthalazine

1g of 6-(4-chlorophenoxy)-tetrazolo[5,1-*a*]phthalazine was added into 120ml of a mixed solvent of nitromethane and water (volume ratio: 2:1), followed by mixing homogeneously until 6-(4-chlorophenoxy)-tetrazolo[5,1-*a*]phthalazine was dissolved. The mixture was volatilized slowly at 50 °C until dry to give 0.96 g of a white crystalline solid (crystalline form D), with a yield of 96%.

### Example 7: Stability of the crystalline forms (suspension balance)

20mg of crystalline form A and 20mg of crystalline form B were mixed, then placed into 1ml of methanol as a solvent, followed by mixing the resultant suspension with a magnetic stirrer at a rotational speed of 100 rpm for two days. And then, the suspension was filtered, and the product obtained was washed with a small amount of methanol, followed by drying at room temperature to give a solid, which was analyzed with XRPD to decide the crystalline form of the product. Other sample mixtures were also shown in Table 1 (see Table 1 for results).

**Table 1 Comparison of the stability of the crystalline forms (suspension balance)**

| Starting sample mixture | Composition of the solvent for suspension | Suspension time (day) | Crystalline form of the product |
|---|---|---|---|
| Form A + Form B | Methanol | 2 | Form A |
| Form A + Form C | Methyl ethyl ketone | 2 | Form A |
| Form A + Form D | Nitromethane + n-hexane | 2 | Form A |
| Form B + Form C | Methyl ethyl ketone | 2 | Form A |
| Form B + Form D | Nitromethane + n-hexane | 2 | Form A |
| Form C + Form D | Methyl ethyl ketone | 2 | Form A |

### Example 8: Comparison of solubility of the crystalline forms

10mg of 6-(4-chlorophenoxy)-tetrazolo[5,1-*a*]phthalazine in each of the four crystalline forms was dissolved into 1ml of water, respectively, and the mixture was stirred for 1 hour so as to reach a solubility equilibrium with a supersaturated state being kept, and then the resultant mixture was filtered to give a clear and saturated water solution for each of the crystalline forms, respectively. Then, each solution was diluted 10 times, and absorbance thereof was measured using a ultraviolet spectrophotometer, and then the concentration ratio of 6-(4-chlorophenoxy)-tetrazolo[5,1-*a*]phthalazine in each solution could be determined according to the Lambert-Beer law, so as to obtain the solubility relationship among the four crystalline forms (see Table 2 for results).

**Table 2 Comparison of solubility of the crystalline forms**

| Crystalline form | Absorbance (Abs) | Solubility relationship with respect to Form A |
|---|---|---|
| Form A | 0.0407 | -- |
| Form B | 0.279 | 6.8 times |
| Form C | 0.326 | 8.0 times |
| Form D | 0.134 | 3.3 times |

### Example 9: Comparison of the pharmacological effect of the anti-convulsion of the crystalline forms

Maximal electroshock (MES) test is an ideal animal model for evaluating a compound having a potential anti-epileptic effect, and if the compound can be applied against the maximal electroshock seizure, then it is likely to be further developed as a drug for treatment of grand mal clinically. Each of the four crystalline forms of this invention was intragastrically (i.g.) administered to mice respectively, each median effective dose (ED₅₀) for the effect against electroshock seizure was measured, and each anti-convulsion effect was assessed.

**Table 3 Comparison of the pharmacological effect of the anti-convulsion of the crystalline forms (mg/kg, p.o)**

| Drug | ED₅₀, MES |
|---|---|
| Crystalline form A | 15.6 |
| Crystalline form B | 9.2 |
| Crystalline form C | 8.5 |
| Crystalline form D | 12.7 |
| Carbamazepine | 15.4 |

The experimental results are reported in Table 3, and the lower the ED₅₀ is, the stronger the anti-convulsion effect is, whereas the higher the ED₅₀ is, the weaker the anti-convulsion effect is. The order of the anti-convulsion effect of the four crystalline forms is as follows: crystalline form C > crystalline form B > crystalline form D > crystalline form A. According to the results of Example 8, crystalline form C had the best water solubility, and when intragastrically administered, it had a high dissolution in stomach, was easy to absorb, and had a high bioavailability; and thus it showed the strongest anti-convulsion effect. Therefore, crystalline form C can be used as a preferred pharmaceutical crystalline form in anti-epileptic application.

It is to be understood that the examples are used to illlustrate the present invention only, and are not intended as limiting the scope of the present invention.

## Claims

1. A crystalline form C of 6-(4-chlorophenoxy)-tetrazolo[5,1-*a*]phthalazine, **characterized by** the following properties:
i) an X-ray powder diffraction pattern having characteristic peaks expressed in degrees 2θ at 8.24, 9.60, 10.07, 11.58, 12.27, 12.74, 13.69, 14.25, 15.17, 16.73, 17.45, 17.92, 18.72, 19.16, 20.35, 21.53, 22.12, 23.37, 23.96, 25.05, 26.62, 28.78, 29.23, 33.84.

2. The crystalline form C of 6-(4-chlorophenoxy)-tetrazolo[5,1-*a*]phthalazine according to claim 1, **characterized by** an X-ray powder diffraction pattern in accordance with Figure 13.

3. A process for preparing the crystalline form C of 6-(4-chlorophenoxy)-tetrazolo[5,1-*a*]phthalazine according to claims 1 or 2, comprising the following steps:
adding 6-(4-chlorophenoxy)-tetrazolo[5,1-*a*]phthalazine into a solvent in the proportion of 15:1 g/L, mixing homogeneously until 6-(4-chlorophenoxy)-tetrazolo[5,1-*a*]phthalazine is dissolved, then volatilizing slowly at a temperature of 25 °C or 50 °C until dry, and then collecting solid to obtain a crystalline form C of 6-(4-chlorophenoxy)-tetrazolo[5,1-*a*]phthalazine;
wherein the solvent along with the corresponding temperature for the volatilizing step is one selected from methyl ethyl ketone along with 25 °C, nitromethane along with 25 °C, methylbenzene along with 25 °C, propanone along with 50 °C, acetonitrile along with 50 °C.

4. Use of the crystalline form C of 6-(4-chlorophenoxy)-tetrazolo[5,1-*a*]phthalazine according to claims 1 or 2 for the manufacturing of an anti-epileptic medicament.

5. A pharmaceutical composition comprising the crystalline form C of 6-(4-chlorophenoxy)-tetrazolo[5,1-*a*]phthalazine according to claims 1 or 2, and a pharmaceutically acceptable excipient.

6. A crystalline form C of 6-(4-chlorophenoxy)-tetrazolo[5,1-*a*]phthalazine according to claims 1 or 2 for use in treating epilepsy.

## Patentansprüche

1. Kristalline Form C von 6-(4-Chlorphenoxy)-tetrazolo[5,1-*a*]phthalazin, **gekennzeichnet durch** folgende Eigenschaften:
i) ein Pulver-Röntgenbeugungsmuster mit charakteristischen Peaks, ausgedrückt in Graden von 2θ bei 8,24, 9,60, 10,07, 11,58, 12,27, 12,74, 13,69, 14,25, 15,17, 16,73, 17,45, 17,92, 18,72, 19,16, 20,35, 21,53, 22,12, 23,37, 23,96, 25,05, 26,62, 28,78, 29,23, 33,84.

2. Die kristalline Form C von 6-(4-Chlorphenoxy)-tetrazolo[5,1-*a*]phthalazin nach Anspruch 1, **gekennzeichnet durch** ein Pulver-Röntgenbeugungsmuster gemäß Figur 13;

3. Verfahren zum Herstellen der kristallinen Form C von 6-(4-Chlorphenoxy)-tetrazolo[5,1-a]phthalazin nach Anspruch 1 oder 2, umfassend folgende Schritte:
Zugabe von 6-(4-Chlorphenoxy)-tetrazolo[5,1-a]-phthalazin in ein Lösungsmittel im Verhältnis von 15:1 g/l; homogen mischen, bis 6-(4-Chlorphenoxy)-tetrazol[5,1-a]phthalazin gelöst ist; dann langsam bei einer Temperatur von 25°C oder 50°C bis zur Trockene verflüchtigen, und dann Sammeln des Feststoffes, um eine kristalline Form C von 6-(4-Chlorphenoxy)-tetrazolo[5,1-a]phthalazin zu erhalten;
wobei das Lösungsmittel zusammen mit der entsprechenden Temperatur für den Verflüchtigungsschritt ausgewählt ist aus Methylethylketon bei 25°C, Nitromethan bei 25°C, Methylbenzol bei 25°C, Propanon bei 50°C, Acetonitril bei 50°C.

4. Verwendung der kristallinen Form C von 6-(4-Chlorphenoxy)-tetrazolo[5,1-a]phthalazin nach Anspruch 1 oder 2 zum Herstellen eines Antiepileptikums.

5. Pharmazeutische Zusammensetzung, umfassend die kristalline Form C von 6-(4-Chlorphenoxy)-tetrazolo[5,1-a]phthalazin nach einem der Ansprüche 1 oder 2, und einen pharmazeutisch annehmbaren Exzipienten.

6. Kristalline Form C von 6-(4-Chlorphenoxy)-tetrazolo[5,1-a]phthalazin nach einem der Ansprüche 1 oder 2 zur Behandlung von Epilepsie.

## Revendications

1. Forme cristalline C de 6-(4-chlorophénoxy)-tétrazolo[5,1-*a*]phthalazine, **caractérisée par** les propriétés suivantes :
i) un diagramme de diffraction de rayons X sur poudre ayant des pics caractéristiques exprimés en degrés 2θ à 8,24, 9,60, 10,07, 11,58, 12,27, 12,74, 13,69, 14,25, 15,17, 16,73, 17,45, 17,92, 18,72, 19,16, 20,35, 21,53, 22,12, 23,37, 23,96, 25,05, 26,62, 28,78, 29,23 et 33,84.

2. Forme cristalline C de 6-(4-chlorophénoxy)-tétrazolo[5,1-*a*]phthalazine selon la revendication 1, **caractérisée par** un diagramme de diffraction de rayons X sur poudre conformément à la figure 13.

3. Procédé de préparation de la forme cristalline C de 6-(4-chlorophénoxy)-tétrazolo[5,1-*a*]phthalazine selon les revendications 1 ou 2, comprenant les étapes suivantes :
ajouter de la 6-(4-chlorophénoxy)-tétrazolo[5,1-*a*]phthalazine à un solvant dans la proportion de 15:1 g/L, mélanger de manière homogène jusqu'à ce que la 6-(4-chlorophénoxy)-tétrazolo[5,1-a]phthalazine soit dissoute, puis la volatiliser lentement à une température de 25 °C ou 50 °C jusqu'à obtenir un produit sec, puis collecter des matières solides pour obtenir une forme cristalline C de 6-(4-chlorophénoxy)-tétrazolo[5,1-*a*]phthalazine ;
le solvant à la température correspondante pour l'étape de volatilisation étant un composé sélectionné parmi la méthyléthylcétone à 25 °C, le nitrométhane à 25 °C, le méthylbenzène à 25 °C, la propanone à 50 °C et l'acétonitrile à 50 °C.

4. Utilisation de la forme cristalline C de la 6-(4-chlorophénoxy)-tétrazolo[5,1-*a*]phthalazine selon les revendications 1 ou 2 dans la fabrication d'un médicament anti-épileptique.

5. Composition pharmaceutique comprenant la forme cristalline C de la 6-(4-chlorophénoxy)-tétrazolo[5,1-*a*]phthalazine selon les revendications 1 ou 2, et un excipient pharmaceutiquement acceptable.

6. Forme cristalline C de la 6-(4-chlorophénoxy)-tétrazolo[5,1-*a*]phthalazine selon les revendications 1 ou 2 destinée à être utilisée dans le traitement de l'épilepsie.
